# FASCICULE DE BREVET EUROPEEN

(11) **EP 4 272 707 B1**
(45) Date de publication et mention de la délivrance du brevet: **19.03.2025**
(21) Numéro de dépôt: 23169071.0
(22) Date de dépôt: 20.04.2023
(51) Int. Cl.: A61F 2/28

(54) **DISPOSITIF FORMANT BARRIERE POUR LA REGENERATION OSSEUSE**
BARRIEREVORRICHTUNG ZUR KNOCHENREGENERATION
BARRIER DEVICE FOR BONE REGENERATION

(30) Priorité: 02.05.2022 FR 2204158
(43) Date de publication de la demande: 08.11.2023
(73) Titulaire: Khoury, Georges, 75016 Paris (FR)
(72) Inventeur: Khoury, Georges, 75016 Paris (FR)
(74) Mandataire: Eidelsberg, Olivier Nathan

(56) Documents cités:
- WO-A1-2011/125760
- FR-A1- 2 713 090
- US-A1- 2013 274 819
- TOMÁS SUCHY, KAREL BALÍK,MIROSLAV SOCHOR, JOSEF PROKOP,JAN BENES: "Radiolucent composites providing high resistance against sterilization decomposition", CERAMICS SILIKATY, vol. 55(4), 16 November 2011 (2011-11-16), Prague, pages 401 - 409, XP002808031, ISSN: 1804-5847, Retrieved from the Internet <URL:https://www.researchgate.net/publication/285821347_Radiolucent_composites_providing_high_resistance_against_sterilization_decomposition> [retrieved on 20221121]

## Description

La présente invention se rapporte à un dispositif destiné à former une barrière en forme de dôme ou coque, notamment dans le domaine de la régénération osseuse, qui permet de créer et protéger un volume au-dessus de la surface d'un os résiduel pour y disposer un élément en biomatériau, qui en se minéralisant sous la protection du dôme vis-à-vis des autres tissus, va former de l'os nouveau dans le volume protégé par le dôme en s'agrégant à la surface de l'os résiduel.

On connait déjà dans l'art antérieur, par exemple de FR2713090A1, une coque de ce genre sous la forme d'un tube hémicylindrique réalisé en titane, qui est maintenu au-dessus d'une surface osseuse à laquelle on souhaite adjoindre du tissu osseux par régénération par deux joints de bordure ou un joint périphérique faisant office de moyen de maintien de la coque à distance de la surface sur laquelle on souhaite régénérer de l'os, l'espace à l'intérieur de la coque étant protégé du tissu gingival.

La présente invention vise à améliorer les dispositifs de l'art antérieur en mettant à disposition un dispositif formant barrière du genre mentionné ci-dessus, qui permet d'améliorer encore plus la précision du volume d'os régénéré, pour ainsi obtenir un os nouveau correspondant le plus précisément possible à ce que le chirurgien souhaite obtenir au départ.

Suivant un premier aspect de l'invention, un dispositif pour la régénération osseuse destiné à former une barrière pour protéger un espace au-dessus d'une surface d'un os, par exemple des maxillaires, pour permettre, après insertion d'un biomatériau qui en se minéralisant va former de l'os nouveau au-dessus de l'os déjà présent, comportant un élément formant barrière auto portant sous la forme d'un dôme ou d'une coque et des moyens de fixation de l'élément formant barrière au-dessus de la surface de l'os pour ainsi former un volume où le biomatériau peut être disposé pour se minéraliser et former de l'os nouveau se greffant à l'os préalablement présent, est caractérisé en ce que l'élément formant barrière en forme de dôme ou coque est en un matériau, notamment non résorbable, tel que l'élément formant barrière est radio-transparent, c'est-à-dire notamment laisse passer les rayons X avec un coefficient de transmission d'au moins 60%, de préférence au moins 70 %, de préférence 80%, encore plus préférablement 90%, notamment sensiblement 100%.

En prévoyant ainsi un matériau suffisamment transparent aux rayons X, le chirurgien peut voir avec suffisamment de précision le volume intérieur formé entre l'élément barrière et la surface de l'os auquel on souhaite adjoindre de la matière osseuse, et ainsi conformer avec précision ce volume, pour l'adapter autant que possible à la forme qu'il souhaite donner à l'implant final.

De préférence, et suivant un autre aspect de l'invention qui constitue en soi une invention, mais qui peut, de manière avantageuse être mis en œuvre en combinaison avec le premier aspect, l'élément barrière est autoportant mais est déformable élastiquement, notamment en compression et/ou en flexion.

De préférence, le dispositif de régénération osseuse forme une barrière pour protéger de manière hermétique l'espace au-dessus d'une surface d'un os, par exemple des maxillaires.

En particulier, l'élément barrière est déformable élastiquement en compression, cette capacité de compression permettant d'obtenir une meilleure formation osseuse.

On peut ainsi conformer le volume dans lequel on souhaite que de la matière osseuse se régénère de la manière la plus précise possible et ainsi obtenir un implant ayant la forme la plus proche possible de ce que souhaite obtenir le chirurgien au début de son intervention. En outre, l'élément barrière peut facilement être réalisé par impression 3D.

En particulier, le biomatériau en lequel est formé l'élément barrière a un module de Young compris entre 2 et 20 GPa, notamment entre 2 et 15 GPa, plus particulièrement entre 2 et 5 GPa, notamment entre 3 et 4 GPa.

Suivant un mode de réalisation préféré de l'invention, le matériau a une résistance à la flexion comprise entre 100 et 200 MPa, notamment entre 150 et 190 MPa, notamment égale sensiblement à 180 MPa.

Suivant un mode de réalisation préféré de l'invention, le matériau de l'élément barrière a une résistance à la traction comprise entre 50 et 120MPa, notamment comprise entre 80 et 100 MPa.

Les valeurs ci-dessus de résistances et de module Young sont données à la température ambiante, c'est-à-dire entre 20 et 25 ° C, notamment à 23°C et sous la pression atmosphérique.

Suivant un mode de réalisation particulièrement préféré, le matériau de l'élément barrière non résorbable est un polyaryléthercétone (PAEK), notamment un polyétheréthercétone (PEEK) ou un polyéthercétonecétone (PEKK).

D'autres exemples de matériaux appropriés incluent l'acide polylactique (PLA) et l'acide polyglycolique (PGA), qui sont résorbables.

Suivant un mode de réalisation préféré de l'invention, l'élément formant barrière comporte au moins une glissière en forme de fente, notamment deux glissières en forme de fentes, de préférence quatre glissières en formes de fentes latérales qui s'étendent dans la direction perpendiculaire à l'ouverture à l'opposé du sommet de la barrière en forme de dôme ou coque et une ou des vis d'ostéosynthèse respectives correspondantes, disposées dans une fente glissière respective permettant une fixation de l'élément formant barrière autour de la surface de l'os à régénérer, le déplacement des vis dans les glissières permettant de régler le volume protégé par l'élément formant barrière et maintenir la pression de l'élément formant barrière sur le biomatériau, pression obtenue grâce à la déformation en compression possible de l'élément formant barrière.

De préférence, le dispositif de régénération osseuse forme une barrière pour protéger de manière hermétique l'espace au-dessus d'une surface d'un os, par exemple des maxillaires.

A titre d'exemple, on décrit un mode de réalisation de l'invention, en se rapportant aux dessins, dans lesquels :
La figure 1 est une vue en perspective d'un élément barrière suivant l'invention en forme de dôme ;
La figure 2 est une vue en coupe schématique de l'élément formant barrière de la figure 1 disposé au-dessus d'un os avant son installation ; et
la figure 3 est une vue similaire à celle de la figure 2 dans la position installée de la barrière.

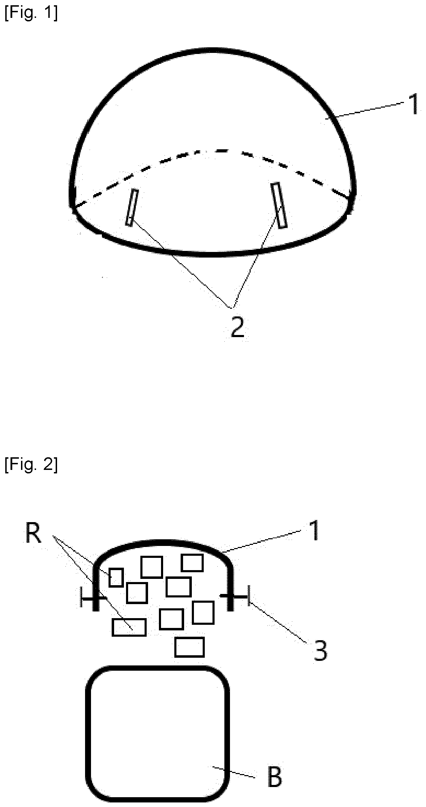

A la figure 1, il est représenté un dôme 1 formé en PEEK ayant une épaisseur de 0,5mm, cette épaisseur pouvant notamment être comprise entre 0,1mm et 1mm. Quatre fentes glissières 2 s'étendent sensiblement à la verticale, à proximité de l'ouverture du dôme, formée à l'opposé du sommet du dôme. Chacune des fentes glissières 2 reçoit en son sein une vis 3 respective qui peut coulisser dans sa fente respective.

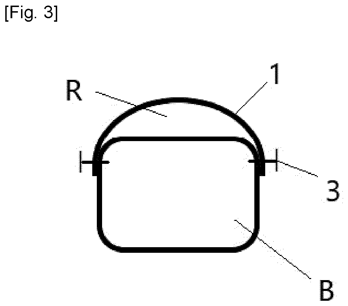

Comme représenté aux figures 2 et 3, le dôme 1 est destiné à venir recouvrir une surface d'un os B en y laissant un volume protégé des autres tissus mous, tels que la gencive ou la peau, ou analogue, pour pouvoir y insérer du biomatériau osseux, qui en se minéralisant sous la protection du dôme vis-à-vis des autres tissus, va former de l'os nouveau dans le volume protégé par le dôme en s'agrégant à la surface de l'os B.

Une fois le volume intérieur du dôme empli du biomatériau R destiné à se développer, le chirurgien peut appliquer une pression sur le dôme pour comprimer le biomatériau qui se trouve en son sein et, à l'aide des vis, fixer le dôme en position finale dans laquelle il maintient une forme (figure 3) sous pression déformée par rapport à sa forme initiale (figure 2).

D'autre part, le chirurgien peut également facilement voir la forme de l'espace intérieur du dôme aux rayons X et l'adapter à la forme de l'implant qu'il souhaite réaliser, tout en pouvant, grâce à la fixation par les vis et au caractère déformable de la paroi du dôme, rajouter ou enlever du biomatériau destiné à former de l'os régénéré.

Comme on le voit à la figure 1 notamment, la paroi du dôme est, de manière préférentielle, pleine, sans ouverture la traversant à l'exception des fentes 2 très fines, notamment de l'ordre de 0,5 à 3 mm en largeur pour une longueur de l'ordre de 2 à 15 mm.

De préférence, la hauteur du dôme, c'est-à-dire la distance perpendiculairement à son ouverture jusqu'à son sommet est fonction du besoin de gain osseux, en général de l'ordre de 3 à 15 mm, dans le domaine dentaire, tandis que cette longueur peut être plus grande, de l'ordre de plusieurs centimètres dans le domaine orthopédique ou maxillofacial.

## Revendications

1. Dispositif pour la régénération osseuse destiné à former une barrière pour protéger un espace au-dessus d'une surface d'un os (B), par exemple d'une dent, pour permettre, après insertion d'un biomatériau qui en se minéralisant va former de l'os nouveau au-dessus de l'os déjà présent, comportant un élément formant barrière auto portant sous la forme d'un dôme (1) ou d'une coque et des moyens de fixation de l'élément formant barrière au-dessus de la surface de l'os pour ainsi former un volume où le biomatériau peut être disposé pour se minéraliser et former de l'os nouveau se greffant à l'os préalablement présent, **caractérisé en ce que** l'élément formant barrière en forme de dôme (1) ou coque est en un matériau, notamment non résorbable, tel que l'élément formant barrière est radio-transparent, c'est-à-dire laisse passer les rayons X avec un coefficient de transmission d'au moins 60%, de préférence au moins 70 %, de préférence 80%, encore plus préférablement 90%, notamment sensiblement 100%.

2. Dispositif suivant la revendication 1, **caractérisé en ce que** l'élément (1) barrière est autoportant mais est déformable élastiquement, notamment en compression et/ou en flexion.

3. Dispositif suivant la revendication 2, **caractérisé en ce que** l'élément (1) barrière est autoportant mais est déformable élastiquement en compression et/ou en flexion.

4. Dispositif suivant la revendication 1, 2 ou 3, **caractérisé en ce que** le matériau en lequel est formé l'élément barrière a un module de Young compris entre 2 et 20 GPa, notamment entre 2 et 15 GPa, plus particulièrement entre 2 et 5 GPa, notamment entre 3 et 4 GPa.

5. Dispositif suivant l'une des revendications 1 à 4, **caractérisé en ce que** le matériau de l'élément barrière a une résistance à la flexion comprise entre 100 et 200 MPa, notamment entre 150 et 190 MPa, notamment égale sensiblement à 180 MPa.

6. Dispositif suivant l'une des revendications 1 à 5, **caractérisé en ce que** le matériau de l'élément barrière a une résistance à la traction comprise entre 50 et 120MPa, notamment comprise entre 80 et 100 MPa.

7. Dispositif suivant l'une des revendications 1 à 6, **caractérisé en ce que** le matériau de l'élément barrière est non résorbable.

8. Dispositif suivant la revendication 7, **caractérisé en ce que** le matériau non résorbable est un polyaryléthercétone (PAEK).

9. Dispositif suivant la revendication 8, **caractérisé en ce que** l'élément barrière est un PAEK sous la forme d'un polyétheréthercétone (PEEK) ou un polyéthercétonecétone (PEKK).

10. Dispositif suivant l'une des revendications 1 à 6, **caractérisé en ce que** le matériau de l'élément barrière est résorbable.

11. Dispositif suivant la revendication 10, **caractérisé en ce que** le matériau résorbable est l'acide polylactique (PLA) et/ou l'acide polyglycolique (PGA).

12. Dispositif suivant l'une des revendications 1 à 11, **caractérisé en ce que** l'élément (1) barrière comporte au moins une glissière (2) en forme de fente, notamment deux glissières en forme de fentes, de préférence quatre glissières en formes de fentes latérales qui s'étendent dans la direction perpendiculaire à l'ouverture à l'opposé du sommet de la barrière en forme de dôme ou coque et une ou des vis (3) d'ostéosynthèse respectives correspondantes, disposées dans une fente glissière respective permettant une fixation de l'élément formant barrière autour de la surface de l'os à régénérer, le déplacement des vis dans les glissières permettant de régler le volume protégé par l'élément formant barrière, grâce à la déformation possible de l'élément formant barrière.

13. Dispositif suivant l'une des revendications précédentes, **caractérisé en ce que** le dispositif de régénération osseuse forme une barrière pour protéger de manière hermétique l'espace au-dessus d'une surface d'un os, par exemple des maxillaires.

## Patentansprüche

1. Vorrichtung zur Knochenregeneration zur Bildung einer Barriere zum Schutz eines Raums über einer Oberfläche eines Knochens (B), z.B. eines Zahns, um nach Einsetzen eines Biomaterials, das durch Mineralisierung neuen Knochen über dem bereits vorhandenen Knochen bildet, die Bildung von neuem Knochen zu ermöglichen, mit einem sich selbst tragenden Barriereelement in Form einer Kuppel (1) oder einer Schale und einer Einrichtung zur Befestigung des Barriereelements über der Knochenoberfläche, um dadurch eine Raum zu bilden, in dem das Biomaterial angeordnet werden kann, um zu mineralisieren und neuen Knochen zu bilden, der auf den bereits vorhandenen Knochen aufgepfropft wird, **dadurch gekennzeichnet, dass** das Barriereelement in Form einer Kuppel (1) oder Schale aus einem insbesondere nicht resorbierbaren Material besteht, so dass das Barriereelement strahlendurchlässig ist, d.h. insbesondere Röntgenstrahlen mit einem Transmissionskoeffizienten von mindestens 60%, vorzugsweise mindestens 70%, vorzugsweise 80%, noch bevorzugter 90%, insbesondere im Wesentlichen 100%, passieren lässt.

2. Vorrichtung nach Anspruch 1, **dadurch gekennzeichnet, dass** das Barriereelement (1) selbsttragend ist, aber elastisch verformbar ist, insbesondere bei Druck und/oder Biegung.

3. Vorrichtung nach Anspruch 2, **dadurch gekennzeichnet, dass** das Barriereelement (1) selbsttragend ist, aber elastisch verformbar ist, und zwar in Druck und/oder Biegung.

4. Vorrichtung nach Anspruch 1, 2 oder 3, **dadurch gekennzeichnet, dass** das Material, aus dem das Barriereelement gebildet ist, einen Elastizitätsmodul zwischen 2 und 20 GPa, insbesondere zwischen 2 und 15 GPa, vor allem zwischen 2 und 5 GPa, insbesondere zwischen 3 und 4 GPa, aufweist.

5. Vorrichtung nach einem der Ansprüche 1 bis 4, **dadurch gekennzeichnet, dass** das Material des Barriereelements eine Biegefestigkeit zwischen 100 und 200 MPa, insbesondere zwischen 150 und 190 MPa, insbesondere im Wesentlichen 180 MPa aufweist.

6. Vorrichtung nach einem der Ansprüche 1 bis 5, **dadurch gekennzeichnet, dass** das Material des Barriereelements eine Zugfestigkeit zwischen 50 und 120 MPa, insbesondere zwischen 80 und 100 MPa aufweist.

7. Vorrichtung nach einem der Ansprüche 1 bis 6, **dadurch gekennzeichnet, dass** das Material des Barriereelements nicht resorbierbar ist.

8. Vorrichtung nach Anspruch 7, **dadurch gekennzeichnet, dass** das nicht resorbierbare Material ein Polyaryletherketon (PAEK) ist.

9. Vorrichtung nach Anspruch 8, **dadurch gekennzeichnet, dass** das Barriereelement ein PAEK in der Form eines Polyetheretherketons (PEEK) oder eines Polyetherketonketons (PEKK) ist.

10. Vorrichtung nach einem der Ansprüche 1 bis 6, **dadurch gekennzeichnet, dass** das Material des Barriereelements resorbierbar ist.

11. Vorrichtung nach Anspruch 10, **dadurch gekennzeichnet, dass** das resorbierbare Material Polymilchsäure (PLA) und/oder Polyglykolsäure (PGA) ist.

12. Vorrichtung nach einem der Ansprüche 1 bis 11, **dadurch gekennzeichnet, dass** das Barriereelement (1) mindestens eine schlitzförmige Gleiteinrichtung (2), insbesondere zwei schlitzförmige Gleiteinrichtungen, vorzugsweise vier seitliche schlitzförmige Gleiteinrichtungen, die sich in der Richtung senkrecht zur Öffnung gegenüber der Spitze der kuppel- oder schalenförmigen Barriere erstrecken, und eine oder mehrere entsprechende jeweilige Osteosyntheseschraube(n) (3) aufweist, die in einer jeweiligen GleitscGleiteinrichtung angeordnet sind, die eine Befestigung des Barriereelements um die Oberfläche des zu regenerierenden Knochens ermöglicht, wobei die Bewegung der Schrauben in der Gleiteinrichtung eine Einstellung des durch das Barriereelement geschützten Volumens durch die mögliche Verformung des Barriereelements ermöglicht.

13. Vorrichtung nach einem der vorhergehenden Ansprüche, **dadurch gekennzeichnet, dass** die Knochenregenerationsvorrichtung eine Barriere bildet, um den Raum über einer Oberfläche eines Knochens, z.B. des Kieferknochens, hermetisch zu schützen.

## Claims

1. A device for bone regeneration intended to form a barrier for protecting a space above a surface of a bone (B), for example a tooth, to allow, after insertion of a biomaterial, which, while mineralising, will form new bone above existing bone, comprising a self-supporting barrier-forming element in the shape of a dome (1) or a shell and means for attaching the barrier-forming element above the bone surface in order thus to form a volume where the biomaterial can be arranged to mineralise and form new bone grafting itself onto the previously present bone, **characterised in that** the barrier-forming element in the shape of a dome (1) or shell is made of a material, in particular a non-resorbing material, such that the barrier-forming element is radiotransparent, i.e. allows through X-rays with a transmission coefficient of at least 60%, preferably at least 70 %, preferably 80%, even more preferably 90%, specifically substantially 100%.

2. The device according to claim 1, **characterised in that** the barrier element (1) is self-supporting but can be deformed elastically, in particular by compression and/or flexion.

3. The device according to claim 2, **characterised in that** the barrier element (1) is self-supporting but can be deformed elastically by compression and/or flexion.

4. The device according to claim 1, 2 or 3, **characterised in that** the material from which the barrier element is made has a Young's modulus of between 2 and 20 GPa, specifically between 2 and 15 GPa, more particularly between 2 and 5 GPa, specifically between 3 and 4 GPa.

5. The device according to one of claims 1 to 4, **characterised in that** the material of the barrier element has a flexural strength of between 100 and 200 MPa, specifically between 150 and 190 MPa, in particular substantially equal to 180 MPa.

6. The device according to one of claims 1 to 5, **characterised in that** the material of the barrier element has a tensile strength of between 50 and 120 MPa, specifically between 80 and 100 MPa.

7. The device according to one of claims 1 to 6, **characterised in that** the material of the barrier element is non-resorbing.

8. The device according to claim 7, **characterised in that** the non-resorbing material is a polyaryletherketone (PAEK).

9. The device according to claim 8, **characterised in that** the barrier element is a PAEK in the form of a polyetheretherketone (PEEK) or a polyetherketoneketone (PEKK).

10. The device according to one of claims 1 to 6, **characterised in that** the material of the barrier element is resorbing.

11. The device according to claim 10, **characterised in that** the resorbing material is polylactic acid (PLA) and/or polyglycolic acid (PGA).

12. The device according to one of claims 1 to 11, **characterised in that** the barrier element (1) comprises at least one runner (2) in the shape of a slit, specifically two runners in the shape of slits, preferably four runners in the shape of lateral slits which extend in the direction perpendicular to the opening opposite the top of the barrier in the shape of a dome or shell and one or some corresponding respective osteosynthesis screw(s) (3), arranged in a respective slit runner allowing the barrier-forming element to be attached around the surface of bone to be regenerated, the movement of the screws in the runners allowing the volume protected by the barrier-forming element to be controlled, due to the possible deformation of the barrier-forming element.

13. The device according to one of the preceding claims, **characterised in that** the device for bone regeneration forms a barrier for protecting, hermetically, the space above a surface of a bone, for example jaws.
